# EUROPÄISCHE PATENTSCHRIFT

(11) **EP 0 579 079 B1**
(45) Veröffentlichungstag und Bekanntmachung des Hinweises auf die Patenterteilung: **22.01.1997**
(21) Anmeldenummer: 93110701.5
(22) Anmeldetag: 05.07.1993
(51) Int. Cl.: A61K 7/42

(54) **Kosmetisches Pflegemittel gegen lichtbedingte Hautalterung**
Cosmetic preparation for treating light-induced skin ageing
Composition cosmétique pour le traitement du photo-vieillissement de la peau

(30) Priorität: 16.07.1992 DE 4223463
(43) Veröffentlichungstag der Anmeldung: 19.01.1994
(73) Patentinhaber: Sara Lee/DE N.V., 3532 AA Utrecht (NL)
(72) Erfinder: Voss, Eckart, Dr., D-51375 Leverkusen (DE); Finkel, Peter, Dipl.-Ing., D-51061 Köln (DE)
(74) Vertreter: Hatzmann, Marinus Jan

(56) Entgegenhaltungen:
- EP-A- 0 343 444
- US-A- 5 102 654

## Beschreibung

Die vorliegende Erfindung betrifft ein neues kosmetisches Pflegepräparat zur äußeren Anwendung auf der Basis von UV-Strahlung absorbierenden Substanzen in Kombination mit einem Radikalfängersystem.

Kosmetische Hautpflegemittel enthalten üblicherweise neben pflegenden Bestandteilen UV-B- und/oder UV-A-Filter zur Reduktion der für die Hautalterung verantwortlichen UV-Strahlen. Dennoch gelangt ein großer Teil dieser Strahlen in tiefe Hautschichten und schädigt diese auf vielfältige Weise. Der bedeutendste Schädigungsmechanismus ist die Bildung von freien Radikalen, sehr energiereichen Zwischenprodukten, die in völlig unselektiver Weise gerade die Hautbestandteile angreifen, die für Elastizität und Feuchtigkeitsbindung verantwortlich sind.

Vitamin-E-Derivate gehören zu den wirksamsten Radikalfängern überhaupt, deren biologische Wirkung auf der Antioxidans-Wirkung der Verbindungen selber beruht (H. Möller et al. Parfümerie und Kosmetik 68, 11, 688 (1987). Die Funktion von üblichen Vitamin-E-Derivaten als Radikalfänger in kosmetischen Hautpflegemitteln wird jedoch durch die unzureichende und/oder inhomogene Verteilung in der Epidermis limitiert. So sind normalerweise gerade in der sensiblen Basalschicht der Epidermis nur relativ geringe Wirkstoffspiegel erzielbar, und selbst mit den heute vielfach eingesetzten Liposomen als Vehikel wird keine große Verbesserung in dieser Hinsicht erzielt.

Es wurde nun ein neues kosmetisches Pflegemittel gegen lichtbedingte Hautalterung gefunden, das neben üblichen Grund- und Hilfsstoffen eine Kombination aus 0,3 bis 8 % (Gew.-%) Vitamin-E-linoleat und/oder Vitamin-E-acetat und 2-(Dihydroxyethyl)-2-hydroxy-6,10,14-trimethyl-pentadecan sowie gegebenenfalls wenigstens einen UV-Filterstoff enthält.

Das 2-(Dihydroxyethyl)-2-hydroxy-6,10,14-trimethyl-pentadecan ist als Haar und Hautpflegemittel literaturbekannt, in denen es dazu beiträgt, die Feuchtigkeit der Haare zu erhalten, das Haar vor mechanischen Beschädigungen zu schützen, das Eindringen von Panthenol und Aminosäuren in den Haarschaft zu begünstigen, mattem Haar zu neuem Glanz verhilft, das Feuchthaltevermögen von Haut zu erhöhen und diese glatt und geschmeidig zu halten. (G. Erlemann et al., Seifen, Öle, Fette 117, 10, 379 (1991.)

Über den Einsatz des 2-(Dihydroxyethyl)-2-hydroxy-6,10,14-trimethyl-pentadecan zusammen mit Vitamin-E wird im Beispiel 1 auf Seite 4 der EP-0 343 444 berichtet, ohne daß ein Grund für dessen Einsatz ersichtlich ist. Phytantriol bewirkt eine erhöhte Penetration und/oder Difussion der Vitamin-E-Derivate in die bzw. der Haut.

Weiterhin wurde gefunden, daß man das neue, erfindungsgemäße kosmetische Pflegemittel gegen lichtbedingte Hautalterung bestehend aus pflegemittel-üblichen Grund-und Hilfsstoffen, einer Kombination aus UV-Filter und 0,3 bis 8 % (Gew.-%) Vitamin-E-linoleat und/oder Vitamin-E-acetat, 2-(Dihydroxyethyl)-2-hydroxy-6,10,14-trimethyl-pentadecan sowie gegebenenfalls wenigstens einen UV-Filterstoff erhält, wenn man die Komponenten miteinander vermischt und verrührt und gegebenenfalls anschließend homogenisiert. Vorzugsweise wird die gesamte Herstellung in einer evakuierten Apparatur durchführt, um das Einarbeiten von Luft zu vermeiden.

Die vorliegende Erfindung betrifft auch allgemein die neue Verwendung von 2-(Dihydroxyethyl)-2-hydroxy-6,10,14-trimethyl-pentadecan zur Erhöhung der Penetration und/oder Diffusion von Vitamin-E-Derivaten in die bzw. der Haut, insbesondere in kosmetischen Hautpflegemitteln.

Überraschenderweise zeigt das erfindungsgemäße kosmetische Pflegemittel keine der üblichen Nachteile im Vergleich zu den aus dem Stand der Technik bekannten Pflegemittelformulierungen.

Um den Einsatz von Vitamin-E-Derivaten zu optimieren, wurden nämlich verschiedene Versuche zur Verbesserung der Penetration und Diffusion der Substanz in der Epidermis durchgeführt, und hier zeigte sich, daß eine Kombination von 0,3 bis 8 % (Gew.-%) Vitamin-E-linoleat und/oder Vitamin-E-Acetat mit 2-(Dihydroxyethyl)-2-hydroxy-6,10,14-trimethyl-pentadecan zu einer völlig homogenen Verteilung der Vitamin-E-Derivate im gesamten Bereich der Epidermis führt. Es konnte weiterhin gezeigt werden, daß das erfindungsgemäße Hautpflegemittel ein Depot an Vitamin-E-Derivat in der Haut besonders in den tieferen Bereichen der Epidermis, die bei Alterungsprozessen von Bedeutung sind, aufbaut. Damit ist eine optimale Voraussetzung für den Einsatz eines Radikalfängersystems in lichtexponierter Haut gegeben.

Das erfindungsgemäße kosmetische Hautpflegemittel stellt somit ein hochwirksames System zum Schutz der Haut bei hervorragenden kosmetischen Eigenschaften dar.

Das erfindungsgemäße kosmetische Hautpflegemittel gegen lichtbedingte Hautalterung enthält neben den für Hautpflegemittel üblichen Grund- und Hilfsstoffen bevorzugt
0,3 bis 8 % Vitamin-E-linoleat und/oder Vitamin-E-acetat und
0,1 bis 10 % 2-(Dihydroxyethyl)-2-hydroxy-6,10,14-trimethyl-pentadecan sowie gegebenenfalls
0,1 bis 20 % eines oder mehrerer UV-Filterstoffe.

Besonders bevorzugt enthält das kosmetische Hautpflegemittel gegen lichtbedingte Hautalterung neben den für Hautpflegemittel üblichen Grund- und Hilfsstoffen
0,3 bis 8 % Vitamin-E-linoleat und/oder Vitamin-E-acetat und
0,3 bis 5 % 2-(Dihydroxyethyl)-2-hydroxy-6,10,14-trimethyl-pentadecan sowie gegebenenfalls
0,3 bis 10 % eines oder mehrerer UV-Filterstoffe.

Die oben aufgeführten kosmetischen Pflegemittel enthalten vorzugsweise wenigstens einen UV-Filterstoff in den angegebenen Konzentrationsbereichen.

Als UV-Filterstoffe kommen vorzugsweise alle in der EG-Positivliste genannten UV-absorbierenden Verbindungen infrage, die in der vierzehnten Richtlinie 92/8/EWG der Kommission vom 18. Febr. 1992 veröffentlicht sind.

Dies sind im allgemeinen Benzylidenkampferverbindungen, p-Aminobenzoesäure sowie ihre Derivate, Cinnamate, Benzoxazol-Derivate; Benzophenonderivate und Benzotriazol-Derivate.

Bevorzugt werden als UV-Filter die folgenden Verbindungen eingesetzt:
N-Propoxylierter 4-Aminobenzoesäure-ethylester (Mischung von Isomeren)
Ethoxilierter 4-Aminobenzoesäure-ethylester
4-Aminobenzoesäure-glycerylester
2-Ethylhexyl-4-dimethylaminobenzoat
2-Ethylhexylsalicyclat
4-Methoxizimtsäure-isopentylester (Mischung von Isomeren)
2-Ethylhexyl-4-methoxycinnamat
2-Hydroxy-4-methoxy-4'-methyl-benzophenon [Mexenon (INN)]
2-Hydroxy-4-methoxybenzophenon-5-sulfosäure und Natriumsalz (Sulisobenzon und Natriumsalz)
α-(2-Oxoborn-3-yliden-toluen)-4-Sulfonsäure und ihre Salze
3-(4'-Methylbenzyliden)-d,1-Campfer
3-Benzylidencampfer
4-Isopropyl-dibenzoylmethan
4-Isopropylbenzylsalicyclat
1-(4-tert-Butylphenyl)-3-(4-methoxyphenyl)propan-1,3-dion
2,4,6-Trianilin-(p-carbo-2'-ethylhexyl-1'-oxi)-1,3,5-triazin.

Besonders bevorzugt verwendet man als UV-Filter die folgenden Verbindungen:
2-Ethoxyhexyl-p-(dimethylamino)-benzoat;
2-Ethylhexyl-p-methoxycinnamat;
3-(4'-Methylbenzyliden)-d,1-kampfer;
2-Hydroxy-5-methoxybenzophenon;
2-Hydroxy-4-methoxybenzophenon-5-sulfonsäure;
2-Phenylbenzimidazol-5-sulfonsäure.

Das erfindungsgemäße kosmetische Mittel enthält neben der o.a. Wirkstoffkombination üblicherweise in kosmetischen Mitteln eingesetzte Grund- und Hilfsstoffe, insbesondere Stabilisatoren und Antioxidantien wie Butylhydroxyanisol, Butylhydroxytoluol, EDTA Salze wie Magnesiumsulfat in Mengen von 0,02 bis 5 % u.a.

Zu den Grund- und Hilfsstoffen zählen desweiteren in der Kosmetik übliche Lösungsmittel wie Wasser bis zu 80 %, Monoalkohole, niedrige Polyalkohole mit 1 bis 6 Kohlenstoffatomen oder Mischungen davon, weiterhin Fettkörper, wie mineralische, tierische, oder pflanzliche Öle wie Paraffinöl oder Wachse wie Mikrowachs, Fettsäuren, Fettalkohole, Fettsäureester wie Cetylstearylisononanoat und Isopropylpalmitat, Fettalkoholether, oxethylierte Fettalkohole, Lanolin und Derivate sowie Silikonöle in Mengen von 0,5 bis 50 %, vorzugsweise 0,5 bis 30 %, besonders bevorzugt in Mengen von 5 bis 30 %.

Das erfindungsgemäße kosmetische Pflegemittel enthält gegebenenfalls Emulgatoren in Mengen von 0,1 bis 20 %, bevorzugt in Mengen von 0,2 bis 10 %, wobei es sich um in der Kosmetik üblicherweise verwendete Emulgatoren, insbesondere um nichtionische, anionische, kationische oder amphotere Verbindungen handelt, z.B. Sterole, Polyol-Fettsäureester und -Fettalkoholether, Alkali- und Triethanolaminsalze von Fettsäuren, Natriumcetylstearylsulfat, Tetracylammoniumhalogenide, Phospholipide. Beispiele hierfür sind Glycerinsorbitanfettsäureester, Polyoxyethylenfettsäureester, Alkyltetraglykolether-o-phosphorsäureester.

Ferner können 0,02 bis 5 %, bevorzugt 0,1 bis 2 % Verdickungsmittel und Vergelungsmittel im erfindungsgemäßen Mittel eingesetzt sein, Dazu zählen Polyacrylsäurederivate, Cellulosederivate, Bentonite, Xanthanderivate, Alginate, Guarmehl und Johannisbrotmehl. Beispiele sind Polyacrylsäureamid und Zinkstearat.

Das erfindungsgemäße Präparat kann weitere in kosmetischen Mitteln übliche Stoffe enthalten, Dazu zählen Feuchthaltemittel (0,5 bis 15 %), Farbstoffe, Puffersubstanzen, Konservierungsmittel und Parfümöle in Mengen von 0,01 bis 5,0 %.

Als Feuchthaltemittel seien beispielhaft aufgeführt: Niedrige Polyalkohole wie Glycerin, Propylenglykol, Butylenglykol, Sorbitol, desweiteren die 2-Pyrrolidon-5-carbonsäure und ihr Natriumsalz, Milchsäure und ihre Salze, Harnstoff, Proteine und Proteinderivate wie Collagen, desweiteren Hyaluronsäure u,a.

Als den erfindungsgemäßen kosmetischen Präparaten zuzusetzende Farbstoffe seien beispielhaft aufgeführt:
Farbe C.I. 16255, Farbe C.I. 61570, Farbe C.I. 42051, Farbe C.I. 15985, Farbe C.I. 77492.

Als Konservierungsmittel kommen vorzugsweise in Frage:
2,4-Hexadiensäure (Sorbinsäure und ihre Salze),
4-Hydroxybenzoesäure, ihre Salze und Ester,
3-Acetyl-6-methyl-2,4(3H)-pyrandion (Dehydracetsäure) und seine Salze,
1,1-Methylen-bis-(3-(1-hydroxymethyl-2,4-dioximidazolidin-5-yl)-harnstoff),
Imidazolidinylharnstoff,
2-Phenoxy-ethanol,
Benzylalkohol.

Das erfindungsgemäße kosmetische Hautpflegemittel liegt vorzugsweise als Emulsion (Creme oder Milch) vor, wobei es sich um Öl-in-Wasser- oder Wasser-in-Öl-Emulsionen handeln kann. Die Darstellung erfolgt im allgemeinen durch Mischen und Rühren der Komponenten, gegebenenfalls mit anschließendem Homogenisieren, gegebenenfalls und vorzugsweise in einer evakuierten Apparatur.

Alle Prozentangaben im vorliegenden Text beziehen sich auf Gewichtsprozente, wenn nichts anderes angegeben ist.

Die Erfindung wird im folgenden anhand der Beispiele näher erläutert, ohne daß dies einschränkenden Charakter haben soll.

### Beispiel 1a

| Öl-in-Wasser-Emulsion (pflegende Creme) | | (Angabe in g) |
|---|---|---|
| I. | Polysorbate 60 | 1,5 |
| | Sorbitanstearate (Emulgator) | 0,9 |
| | Octyldodecanol | 10,0 |
| | Cetearyl Alkohol (Fettkomponenten) | 6,0 |
| II. | Polyacrylsäureamid (Verdicker) | 0,6 |
| III. | Vitamin-E-linoleate | 5,0 |
| | 2-(Dihydroxyethyl)-2-hydroxy-6,10,14-trimethyl-pentadecan | 2,0 |
| IV. | Parfümöl | 0,3 |
| | Konservierungsmittel | q.s.* |
| V. | Glycerin (Feuchthaltemittel) | 5,0 |
| | Wasser | ad 100,0 |

| | | |
|---|---|---|
| *⁾ q.s. = quantum satifans, d.h. die in Abhängigkeit vom jeweiligen Konservierungsmittel einzusetzende bzw. ausreichende Menge | | |

### Beispiel 1b

Zusammensetzung wie in Beispiel 1a jedoch zusätzlich 2,0 g p-Methoxyzimtsäure-ethylester als UV-B-Filer bei 66,1 g Wassergehalt.

### Herstellung der Beispiele 1a und 1b:

Die Mischung I wird bei 75°C zum Schmelzen gebracht, II eindispergiert und der auf die gleiche Temperatur erwärmten Lösung V zugesetzt. Unter weiterem Rühren und Homogenisieren läßt man auf 35°C abkühlen, dann gibt man die Mischung III und IV hinzu, ergänzt mit Wasser auf 100 g und läßt unter weiterem Rühren auf Raumtemperatur abkühlen, Die gesamte Herstellung erfolgt in einer evakuierten Apparatur, um das Einarbeiten von Luft zu vermeiden.

### Beispiel 2a

| Öl-in-Wasser-Emulsion (Pflegelotion) | | (Angabe in g) |
|---|---|---|
| I. | Trilaureth-4-phosphate (Emulgator) | 1,0 |
| | Paraffinöl dünnflüsig (Fettsäure-komponenten) | 10,0 |
| | Isopropylpalmitat | 5,0 |
| II. | Polyacrylsäureamid (Verdicker) | 0,8 |
| III. | Vitamin-E-linoleate | 0,5 |
| | 2-(Dihydroxyethyl)-2-hydroxy-6,10,14-trimethyl-pentadecan | 0,5 |
| IV. | Parfümöl | 0,3 |
| | Konservierungsmittel | q.s. |
| V. | Glycerin (Feuchthaltemittel) | 5,0 |
| | Saccharide Isomerate | 3,0 |
| | Wasser | ad 100,0 |

### Beispiel 2b

Zusammensetzung wie Beispiel 2a, jedoch zusätzlich 2,0 g UV-B-Filter bei 71,3 g Wassergehalt.

### Herstellung der Beispiele 2a und 2b:

Die Mischung I wird bei 75°C zum Schmelzen gebracht, II eindispergiert und der auf die gleiche Temperatur erwärmten Lösung V zugesetzt. Unter weiterem Rühren und Homogenisieren läßt man auf 35°C abkühlen, dann gibt man die Mischung III und IV hinzu, ergänzt mit Wasser auf 100 g und läßt unter weiterem Rühren auf Raumtemperatur abkühlen. Die gesamte Herstellung erfolgt in einer evakuierten Apparatur, um das Einarbeiten von Luft zu vermeiden.

### Beispiel 3a

| Wasser-in-Öl-Emulsion (Pflegecreme) | | (Angaben in g) |
|---|---|---|
| I. | Cetyl Dimethicone Copoliol (und) Cetyldimethicon (und) Polyglyceryl-3-oleat (und) Hexyllaurat (Emulgator) | 5,0 |
| | Isohexadecan (Fett-komponenten) | 8,0 |
| | Capryic/Capric Triglyceride | 8,0 |
| | Mikrowachs | 5,0 |
| | p-Methoxyzimtsäureethylester (UV-B-Filter) | 2,0 |
| II. | Vitamin-E-linoleat | 2,0 |
| | 2-(Dihydroxyethyl)-2-hydroxy-6,10,14-trimethyl-pentadecan | 1,0 |
| III. | Parfümöl | 0,5 |
| | Konservierungsmittel | q.s. |
| IV. | Glycerin (Feuchthaltemittel) | 3,0 |
| | Natriumchlorid (Stabilisator) | 1,5 |
| | Wasser | ad 100,0 |

### Beispiel 3b

Zusammensetzung wie Beispiel 3a, jedoch ohne UV-B-Filter bei 65,4 g Wassergehalt.

### Herstellung der Beispiele 3a und 3b:

Die Mischung I wird bei 75°C zum Schmelzen gebracht und die auf die gleiche Temperatur erwärmte Lösung IV unter Rühren zugegeben. Unter weiterem Rühren und Homogenisieren läßt man auf 35°C abkühlen, dann gibt man die Mischung II und III hinzu, ergänzt mit Wasser auf 100 g und läßt unter weiterem Rühren auf Raumtemperatur abkühlen, Die gesamte Herstellung erfolgt in eienr evakuierten Apparatur, um das Einarbeiten von Luft zu vermeiden.

## Patentansprüche

1. Kosmetisches Hautpflegemittel, dadurch gekennzeichnet, daß es neben üblichen Grund- und Hilfsstoffen eine Kombination aus 0,3 bis 8 % (Gew.-%) Vitamin-E-linoleat und/oder Vitamin-E-acetat und 2-(Dihydroxyethyl)-2-hydroxy-6,10,14-trimethyl-pentadecan sowie gegebenenfalls wenigstens einen UV-Filterstoff enthält.

2. Kosmetisches Hautpflegemittel gemäß Anspruch 1, dadurch gekennzeichnet, daß es
0,1 bis 10 % (Gew.-%) 2-(Dihydroxyethyl)-2-hydroxy-6,10,14-trimethyl-pentadecan enthält.

3. Kosmetisches Hautpflegemittel gemäß der Ansprüche 1 und 2, dadurch gekennzeichnet, daß es
0,1 bis 20 % (Gew.-%) eines oder mehrerer UV-Filterstoffe enthält.

4. Kosmetisches Hautpflegemittel in Emulsionsform gemäß Anspruch 1, dadurch gekennzeichnet, daß es neben üblichen Grund- und Hilfsstoffen eine Kombination aus
0,3 bis 10 % (Gew.-%) eines oder mehrerer UV-Filterstoffe,
0,3 bis 8 % (Gew.-%)Vitamin-E-linoleat und/oder Vitamin-E-acetat und
0,3 bis 5 % (Gew.-%) 2-(Dihydroxyethyl)-2-hydroxy-6,10,14-trimethyl-pentadecan enthält.

5. Kosmetisches Hautpflegemittel gemaß den Ansprüche 1 bis 4, dadurch gekennzeichnet, daß es sich um Öl-in-Wasser- oder Wasser-in-Öl-Emulsionen in Milch- oder Creme-Form handelt.

6. Kosmetisches Hautpflegemittel gemäß der Ansprüche 1 bis 5, dadurch gekennzeichnet, daß es als Grund-oder Hilfsstoffe einen oder mehrere der folgenden Stoffe enthält:
Antioxidantien, Lösungsmittel, mineralische, tierische oder pflanzliche Öle oder Wachse, Fettsäuren, Fettalkohole, Fettsäureester, Fettalkoholether, ethoxylierte Fettalkohole, Lanolin oder Lanolinderivate, Silikonöle, Emulgatoren, Verdickungsmittel, Feuchthaltemittel, Farbstoffe, Puffersubstanzen, Konservierungsmittel und Parfümöle.

7. Verfahren zur Herstellung von kosmetischen Hautpflegemitteln gemäß der Ansprüche 1 bis 6, dadurch gekennzeichnet, daß die in Anspruch 1 genannten Wirkstoffe mit den üblichen Grund- und Hilfsstoffen gegebenenfalls unter Vordispergieren, Rühren und/oder Homogenisieren, gegebenenfalls in einer evakuierten Apparatur vermischt werden.

8. Verwendung von 2-(Dihydroxy-ethyl)-2-hydroxy-6,10,14-trimethyl-pentadecan zur Erhöhung der Penetration und/oder Diffusion von Vitamin-E-Derivaten in die bzw. der Haut.

## Claims

1. Cosmetic skin care preparation characterised in that in addition to the usual raw materials and additional substances, it also contains a combination of 0.3% to 8% (% ^{w}/_{w}) of vitamin E linoleate and/or vitamin E acetate and 2-(dihydroxyethyl)-2-hydroxy-6,10,14-trimethyl-pentadecan and, if necessary, at least 1 UV filter substance.

2. Cosmetic skin care preparation in accordance with claim 1, characterised in that it contains 0.1% to 10% (% ^{w}/_{w}) 2-(dihydroxyethyl)-2-hydroxy-6,10,14-trimethyl-pentadecan.

3. Cosmetic skin care preparation in accordance with claims 1 and 2, characterised in that it contains 0.1% to 20% (% ^{w}/_{w}) of one or more UV filter substances.

4. Cosmetic skin care preparation in emulsion form in accordance with claim 1, characterised in that, in addition to the usual raw materials and additional substances, it also contains a combination of
0.3% to 10% (% ^{w}/_{w}) of one or more UV filter substances
0.3% to 8% (% ^{w}/_{w}) vitamin E linoleate and/or vitamin E acetate and
0.3% to 5% (% ^{w}/_{w}) 2-(dihydroxyethyl)-2-hydroxy-6,10,14-trimethyl-pentadecan.

5. Cosmetic skin care preparation in accordance with claims 1 to 4, characterised in that it is an oil in water or water in oil emulsion in milk or cream form.

6. Cosmetic skin care preparation in accordance with claims 1 to 5, characterised in that it contains one or more of the following substances as the raw materials or additional substances:
Antioxidants, solvents, animal, vegetable or mineral oils or waxes, fatty acids, fatty alcohols, fatty acid ester, fatty alcohol ether, ethoxylated fatty alcohols, lanolin or lanolin derivatives, silicon oils, emulsifiers, thickeners, moisture retaining agents, pigments, buffer substances, preservation agents and perfume oils.

7. Process for manufacturing cosmetic skin care preparations in accordance with claims 1 to 6, characterised in that the active ingredients indicated in claim 1 are mixed with the normal raw materials and additional substances, with predispersion, stirring and/or homogenisation if necessary, in an evacuated apparatus if necessary.

8. Use of 2-(dihydroxy-ethyl)-2-hydroxy-6,10,14-trimethyl-pentadecan for increasing the penetration and/or diffusion of vitamin E derivatives in or into the skin.

## Revendications

1. Produit cosmétique de soin de la peau, caractérisé en ce que, en plus des substances de base et substances auxiliaires habituelles, il contient une combinaison de 0,3 à 8% (% en poids) de linoléate de vitamine E et/ou d'acétate de vitamine E et de 2-(dihydroxy-éthyl)-2-hydroxy-6,10,14-triméthyl-pentadécane ainsi que, le cas échéant, au moins une substance filtrant les UV.

2. Produit cosmétique de soin de la peau suivant la revendication 1, caractérisé en ce qu'il contient de 0,1 à 10% (% en poids) de 2-(dihydroxy-éthyl)-2-hydroxy-6,10,14-triméthyl-pentadécane.

3. Produit cosmétique de soin de la peau suivant les revendications 1 et 2, caractérisé en ce qu'il contient 0,1 à 20% (% en poids) d'une ou plusieurs substances filtrant les UV.

4. Produit cosmétique de soin de la peau, sous forme d'émulsion, suivant la revendication 1, caractérisé en ce que, en plus des substances de base et substances auxiliaires habituelles, il contient une combinaison de
0,3 à 10% (% en poids) d'une ou plusieurs substances filtrant les UV,
0,3 à 8% (% en poids) de linoléate de vitamine E et/ou d'acétate de vitamine E et
0,3 à 5% (% en poids) de 2-(dihydroxy-éthyl)-2-hydroxy-6,10,14-triméthyl-pentadécane.

5. Produit cosmétique de soin de la peau suivant les revendications 1 à 4, caractérisé en ce qu'il s'agit d'émulsions d'huile dans l'eau ou d'eau dans l'huile sous forme de lait ou de crème.

6. Produit cosmétique de soin de la peau suivant les revendications 1 à 5, caractérisé en ce qu'il contient comme substances de base et substances auxiliaires une ou plusieurs des substances suivantes : antioxydants, solvants, huiles ou cires minérales, animales ou végétales, acides gras, alcools gras, esters d'acides gras, éthers d'acides gras, alcools gras éthoxylés, lanoline ou dérivés de la lanoline, huiles siliconées, émulsifiants, produits épaississants, produits humidifiants, colorants, substances tampons, agents de conservation et huiles parfumées.

7. Procédé de fabrication de produits cosmétiques de soin de la peau suivant les revendications 1 à 6, caractérisé en ce que les substances actives citées dans la revendication 1 sont mélangées avec les substances de base et substances auxiliaires habituelles, le cas échéant sous dispersion préalable, agitation et/ou homogénéisation, le cas échéant dans un appareillage mis sous vide.

8. Utilisation de 2-(dihydroxy-éthyl)-2-hydroxy-6,10,14-triméthyl-pentadécane afin d'augmenter la pénétration et/ou la diffusion de dérivés de la vitamine E dans ou à l'intérieur de la peau.
